# EUROPÄISCHE PATENTSCHRIFT

(11) **EP 3 183 493 B1**
(45) Veröffentlichungstag und Bekanntmachung des Hinweises auf die Patenterteilung: **10.10.2018**
(21) Anmeldenummer: 15766747.8
(22) Anmeldetag: 21.08.2015
(51) Int. Cl.: F21V 29/58, C12M 1/00, C12M 1/02, C02F 1/32, B01J 19/12, F21V 31/00

(54) **LAMPENMODUL MIT LICHT EMITTIERENDEN DIODEN UND PHOTOREAKTOR**
LAMP MODULE COMPRISING LIGHT-EMITTING DIODES AND PHOTOREACTOR
MODULE DE LAMPE COMPORTANT DES DIODES LUMINESCENTES ET PHOTORÉACTEUR

(30) Priorität: 21.08.2014 DE 102014012217
(43) Veröffentlichungstag der Anmeldung: 28.06.2017
(73) Patentinhaber: Peschl Ultraviolet GmbH, 55130 Mainz (DE)
(72) Erfinder: PESCHL, Alexander, 55130 Mainz (DE)
(74) Vertreter: mepat Patentanwälte
(86) Internationale Anmeldenummer: PCT/EP2015/001713
(87) Internationale Veröffentlichungsnummer: WO 2016/026576

(56) Entgegenhaltungen:
- DE-A1-102010 042 670
- US-A1- 2010 267 125

## Beschreibung

Die Erfindung betrifft ein Lampenmodul mit Licht emittierenden Dioden (LEDs), das als Tauchstrahler in Photoreaktoren zur Durchführung photochemischer Reaktionen oder auch in sogenannten AOPs ("advanced oxidation processes") sowie zu Desinfektionszwecken eingesetzt werden kann, sowie einen entsprechenden Photoreaktor.

Aus dem Stand der Technik ist bekannt, Strahlungsquellen, insbesondere auch UV Strahler als Tauchlampen in einem flüssigen Medium einzusetzen, um z. B. Wasser zu desinfizieren oder um photochemische Reaktionen durchzuführen. Herkömmlicher Weise werden in der Regel Niederdruck- oder Mitteldruckstrahler (Entladungslampen) eingesetzt, die z. B. zum Schutz vor Verschmutzung von einem Tauchrohr umhüllt werden, das für die emittierte bzw. für die photochemische Reaktion relevante Strahlung durchlässig ist. Das Emissionsspektrum der Enlladungslampen kann durch geeignete Dotierung variiert und entsprechend der Anforderungen für die photochemische Reaktion in gewissem Umfang angepasst werden.

Allerdings ist der Einsatz von Niederdruck- oder Mitteldruckstrahler mit hohem Stromverbrauch verbunden und mit zunehmendem Alter lässt die Strahlungsintensität deutlich nach, ferner kann sich das Strahlungsspektrum verschieben, was eine regelmäßige Überprüfung in relativ kurzen Zeitabständen erforderlich macht.

Im Leuchtmittelbereich finden LEDs zunehmend Bedeutung aufgrund ihres vergleichsweise geringen Stromverbrauchs, hoher Lebensdauer und der hohen Schaltfestigkeit bei spontan vollem Lichtstrom. Die LED strahlt Licht, Infrarot- oder UV-Strahlung aus, wenn elektrischer Strom in Durchlassrichtung fließt. Die Wellenlänge der emittierten Strahlung hängt von der Dotierung des Halbleiterbauelements ab. Für UV-Strahlung kommen etwa Diamant, Aluminiumnitrid, Aluminiumgalliumnitrid oder Aluminiumgalliumindiumnitrid in Frage.

LEDs sind zwar keine Wärmestrahler wie etwa Mitteldruckstrahler, allerdings verkürzen hohe Temperaturen - gewöhnlich hervorgerufen durch hohe Ströme, die für eine maximale Lichtausbeute benötigt werden - die Lebensdauer der LEDs deutlich. Um diesen nachteiligen Einfluss der erhöhten Temperatur bei höheren Strömen zu vermeiden, werden LEDs häufig nicht bei Nennleistung sondern darunter - verbunden mit geringerer Leuchtleistung - betrieben. Um dennoch eine gewünschte Lichtmenge zu erreichen, wird dann die Anzahl der eingesetzten LEDs erhöht.

Sollen die LEDs für eine hohe Lichtausbeute bzw. Strahlungsintensität mit hohen Strömen betrieben werden, ist eine effektive Wärmeableitung erforderlich, um die Lebensdauer der LEDs zu erhalten. Häufig werden Metallgehäuse, meistens aus Aluminium, zur Wärmeableitung eingesetzt.

Für den Einsatz von LEDs in Photobioreakloren zur Kultivierung von Mikroorganismen wird in der US2010/0267125 A1 ein LED-Leuchtmodul beschrieben, bei dem die LEDs gekühlt werden. Die LEDs sind auf einem einseitig geschlossenen Trägerrohr angeordnet, das von einem transparenten Hüllrohr umgeben ist. Innerhalb des Trägerrohrs ist ein Innenrohr angeordnet, das oberhalb des geschlossenen Endes des Trägerrohrs endet, sodass innerhalb des Trägerrohrs ein Fluidpfad mit einem stromaufwärts gelegenen Abschnitt in dem Spalt zwischen Trägerrohr und Innenrohr und mit einem stromabwärts gelegenen Abschnitt in dem Innenrohr vorliegt. Der stromaufwärts gelegene Abschnitt ist über ein Kopfteil, das das Träger- und Innenrohr verschließt, fluidisch mit einer Kühlmittelquelle und der stromabwärts gelegene Abschnitt entsprechend mit einem Kühlmittelrücklauf verbunden.

In Bioreaktoren liegen im Reaktionsraum Druck- und Temperaturbedingungen vor, die sich nicht wesentlich von Umgebungsdruck und Raumtemperatur unterscheiden, da nur so üblicherweise ein optimales Wachstum der Mikroorganismen erreicht werden kann.

Sollen LEDs in Photoreaktoren für chemische Synthesen, z B. Photochlorierung oder Photobromierung zum Einsatz kommen, so muss ein entsprechendes Lampenmodul einer Tauchlampe den erhöhten Anforderungen hinsichtlich den umgebenden Reaktionsbedingungen genügen, die sehr deutlich von Umgebungsdruck und Raumtemperatur abweichen können. Zur thermischen Entkopplung einer Strahlungsquelle von dem umgebenden Reaktionsraum ist aus DE 10 2010 042670 A1 ein doppelwandiges Tauchrohr bekannt. Ferner ist bislang der Einsatz von LEDs im industriellen Maßstab, in dem Reaktionsräume auch mehrere Meter durchmessen können und in dem die bislang als Tauchstrahler eingesetzten Niederdruck- oder Mitteldruckstrahler Abmessungen in entsprechender Größenordnung aufweisen, aufgrund der Betriebsweise der LEDs mit geringer Spannung und der für eine hohe Strahlungsleistung erforderlichen hohen Stromstärke nicht möglich.

Ausgehend von diesem Stand der Technik ist es Aufgabe der vorliegenden Erfindung, ein hinsichtlich der Handhabung des Wärmehaushalts verbessertes LED-Lampenmodul bereitzustellen, das nicht nur unter Umgebungsbedingungen und im Labormaßstab, sondern als Tauchrohr zur photochemischen Synthese oder photokatalysierten Synthese, bei AOPs sowie zur Desinfektion, auch im industriellen Maßstab, eingesetzt werden kann.

Diese Aufgabe wird durch ein LED-Lampenmodul mit den Merkmalen des Anspruchs 1 gelöst.

Weiterbildungen des Gegenstands sind in den Unteransprüchen ausgeführt.

Die weitere Aufgabe, einen Photoreaktor mit LEDs als Strahlungsquelle bereitzustellen, wird durch den Photoreaktor mit den Merkmalen des unabhängigen Anspruchs 10 gelöst.

Ein erfindungsgemäßes Lampenmodul, das dazu ausgebildet ist, als Tauchstrahler in photochemischen Reaktoren eingesetzt zu werden, weist einen Kühlkörper auf, an dessen Außenseite zumindest eine Trägerstruktur - meist eine Platine - mit zumindest einer Licht emittierenden Diode (LED) angeordnet ist. Der Kühlkörper begrenzt zumindest einen Fluidpfad, in dem ein Kühlmittel geführt wird bzw. zirkuliert. Welches Kühlmittel geeigneter Weise eingesetzt wird, kann von den Reaktionstemperaturen im umgebenden Reaktionsraum abhängen. Der Fluidpfad gliedert sich in einen oder mehrere Zufuhr- und Rücklaufabschnitte, wobei der oder die Zufuhrabschnitt(e) über ein Kopfteil des Lampenmoduls mit einer Kühlmittelquelle und der oder die Rücklaufabschnitt(e) über das Kopfteil, das ferner zum elektrischen Anschluss der zumindest einen LED und zur Halterung des Lampenmoduls dient, mit einem Kühlmittelrücklauf verbunden sind. Kühlfluidquelle und Rücklauf können auch einen Kreislauf bilden.

Üblicherweise können Lampen zum Schutz vor den Reaktionsbedingungen im umgebenden Reaktionsraum in einem Tauchrohr angeordnet sein. Um die thermisch sensiblen LEDs von den im umgebenden Reaktionsraum herrschenden Bedingungen abzukoppeln, sodass die Funktionsfähigkeit der LEDs nicht beeinflussen wird, weist ein erfindungsgemäßes Lampenmodul entweder zwei (gegebenenfalls auch mehr) ineinander angeordnete Tauchrohre oder zumindest ein doppelwandiges Tauchrohr auf, in dem zumindest der Kühlkörper, der mittels Trägsrstrukturen mit LEDs bestückt ist, gegebenenfalls auch das Kopfteil, angeordnet sind, sodass der zwischen den beiden Tauchrohren oder den Doppelwänden gebildete Spalt eine thermischen Entkopplung bereitstellt. Damit bei längeren LED-Körpern, bei den zwei oder mehr Trägerstrukturen mit LEDs längs des Kühlkörpers aneinander angrenzen, der elektrische Kontakt zu den LEDs auf der Trägerstruktur, die von dem Kopfende beabstandet angeordnet ist, ohne Verschattung der darüber liegenden LED geschaffen werden kann, begrenzt der Kühlkörper neben dem Fluidpfad ferner eine oder mehrere Kammer(n) zumindest teilweise, durch die sich zumindest eine Stromleitungsvorrichtung von dem Kopfende des Kühlkörpers bis zu einem Kontaktelement der LED(s) erstreckt, das an der Trägerstruktur vorliegt.

Die thermische Entkopplkung kann realisiert werden, indem mit einer Absaugvorrichtung ein Unterdruck in dem Spalt erzeugt wird oder indem ein Kühlkreis zur Fluidkühlung in dem Spalt verbunden wird. Bei der Ausführung als doppelwandiges Tauchrohr kann der Spalt zwischen den Wänden auch schon bei dessen Herstellung evakuiert werden. Als Kühlfluid eignen sich alle Medien, die die emittierte Strahlung der LEDs nicht absorbieren, beispielsweise Wasser oder andere Flüssigkeiten. Vorzugsweise kann hier zur Kühlung aber auch eine Gasspülung, insbesondere mit Inertgas oder auch Luft, in Betracht kommen. Vorteilhaft wird durch das zusätzliche Tauchrohr die Oberfläche der Tauchlampe vergrößert und damit wird eine erhöhte photochemische Effizienz realisiert.

Das bzw. die Tauchrohr(e) ist/sind sinnvollerweise aus einem für die Wellenlängen der von den LEDs emittierten Strahlung durchlässigen Material gefertigt. Zudem ist das Tauchrohr hinsichtlich Materialwahl und Formgebung auf Reaktionsdrücke im Bereich von Hochvakuum bis 6 bar Überdruck, die in dem das Tauchrohr umgebenden Reaktionsraum herrschen können, ausgelegt. Als Materialien kommen natürliches oder synthetisches Quarzglas bzw. Quarzglasmischungen oder Borsilikat, etc. in Frage, konstruktiv wird das Tauchrohr mit einer Wandstärke von zumindest 3,5 mm ausgelegt. Ein Konusflansch zur Aufnahme des Kopfteils verbessert die mechanische Stabilität des Lampenmoduls als Tauchlampe weiter. Verwendete Tauchrohre können ferner zumindest einmal getempert und bis zu 10 bar druckgeprüft sein.

Das Tauchrohr kann einseitig geschlossen sein, sodass das geschlossene Ende im Reaktionsraum des umgebenden Photoreaktors liegen kann, wie es häufig bei vertikaler Anordnung von Tauchlampen der Fall ist; das Tauchrohr kann aber auch beidseitig offen sein und auch horizontal in Reaktoren eingesetzt werden, wobei die offenen Enden des Tauchrohrs quasi aus dem Reaktionsraum ragen.

Damit das Lampenmodul auch in Reaktionsumgebungen, die potentiell explosive Atmosphären bilden können, betrieben werden kann, kann vorgesehen sein, dass das Kopfteil an dem offenen Ende eines einseitig geschlossenen Tauchrohrs dicht und federnd gelagert damit verbunden ist. Ein Inertgaszufuhrkanal erstreckt sich durch den Kühlkörper oder als Schlauch oder Rohr durch den zwischen dem Kühlkörper und dem Tauchrohr gebildeten Raum bis oberhalb des geschlossenen Endes des Tauchrohrs. Durch die federnde Lagerung des Kopfteil mit dem Tauchrohr können Vibrationen zur Vermeidung von Glas-Metallkontakt gedämpft werden und durch die abgedichtete Verbindung ein Überdruck In dem Tauchrohr gegenüber dem Umgebungsdruck von zumindest 0,8 mbar bis höchstens 15 mbar erzeugt und aufrecht erhalten werden, sodass keine zündfähige Atmosphäre ins Innere des Tauchrohrs zu dem LED-Körper und dem Kopfteil dringen kann.

Wird ein beidseitig offenes Tauchrohr verwendet, wird das eine Ende durch das abdichtend und federnd gelagerte Kopfteil verschlossen und das andere Ende durch ein entsprechendes Verschlussteil. Die Inertgaszufuhr zur Schaffung des Überdrucks im Tauchrohrinneren kann dann auf einer der beiden Seiten erfolgen.

Die erforderliche Anzahl und/oder Größe der Kammer(n) ist von der Gesamtanzahl der LEDs des Lampenmoduls und deren Verteilung entlang der Kühlkörpermantelfläche abhängig, denn mit zunehmender LED-Zahl wächst der erforderliche Kabelquerschnitt infolge der bei Reihenschaltung von Halbleiterbauteilen auftretenden Kapazitätsverluste, die mit geringer Spannung betrieben werden. Dabei kann eine vorbestimmte Anzahl an LEDs, die ein Bruchteil der Gesamtanzahl sein kann und die bevorzugt auf einer gemeinsamen Trägerstruktur angeordnet sind, jeweils mit einer der elektrischen Leitungen verbunden sein. So kann eine maximale Anzahl an LEDs, die auf einer Trägerstruktur angeordnet werden, und damit eine Maximallänge einer Trägerstruktur vorbestimmt sein.

Der Kühlkörper kann entsprechend der Anzahl und Anordnung der LEDs auf der zumindest einen Trägerstuktur dimensioniert sein. Vorzugsweise entspricht die Anordnung der LEDs auf der bzw. den Trägerstruktur(en), die auf dem Kühlkörper angeordnet ist/sind, einem Verlauf der Rücklaufabschnitte innerhalb des Kühlkörpers, sodass eine effektive Kühlung der LEDs bereitgestellt wird. Der Kühlkörper kann durch ein zylindrisches, vorzugsweise regelmäßig prismatisches Hohl- oder Vollprofil gebildet werden, wobei die Trägerstruktur(en) mit den LEDs auf den ebenen (nicht-konkaven) Abschnitten der Mantelfläche des Prismas angeordnet sind.

Bevorzugte Anordnungen der Zufuhr- und Rücklaufabschnitte des Kühlfluidpfads in dem Kühlkörper können einen längsaxial und zentral verlaufenden Zufuhrabschnitt vorsehen, der sich in mehrere Rücklaufabschnitt verteilt, die axialparallel bzw konzentrisch um den Zufuhrabschnitt entsprechend der Anordnung der Trägerstrukturen mit den LEDs an der Mantelfläche des Kühlkörpers angeordnet sind. Generell kann die Fließrichtung des Fluidpfads aber auch umgekehrt sein, sodass mehrere Zufuhrabschnitte axialparallel bzw konzentrisch um einen längsaxial und zentral verlaufenden Rücklaufabschnitt angeordnet sind.

Die Kammer für die elektrische Leitung kann dann - je nach Fertigungsart den Kühlkörpers und nach Verlauf des Fluidpfads - innerhalb des Kühlkörpers längsaxial und zentral oder konzentrisch um einen längsaxial und zentral verlaufenden Fluidpfadabschnitt verlaufen, beispielsweise in Räumen zwischen dem längsaxial und zentral verlaufenden Zufuhrabschnitt und den Rücklaufabschnilten, wenn etwa der Kühlkörper durch Strangpressprofile gebildet wird. Alternativ können mehrere axialparallel verlaufende Kammern durch Einschnitte des Kühlkörpers an dessen Außenseite in Verbindung mit den an dem Kühlkörper angeordneten Trägerstrukturen gebildet werden, die einen durch die Einschnitte des Kühlkörpers gebildeten Hohlraum verschließen.

An dem Kopfteil des Lampenmoduls können Kühlmittel-, elektrische und mechanische Anschlussvorrichtungen vorgesehen sein. Die elektrische Anschlussvorrichtung sorgt für die Verbindung der Stromversorgungs- und/oder Steuerungsleitung mit einer Stromversorgungs- und Steuerungsvorrichtung des Lampenmoduls. Die Stromversorgungs- und Steuerungsvorrichtung des Lampenmoduls für die LEDs, die beispielsweise Vorschalt-bzw. Leistungselektronik, Treiber und Netzteile umfassen kann, kann gegebenenfalls im Kopfteil untergebracht sein. Zur Kühlung einer im Kopfteil untergebrachten Stromversorgungs- und Steuerungsvorrichtung kann das Kopfteil dann ebenfalls einen oder mehrere Kühlmittelpfad(e) aufweisen, der vorzugsweise über die Kühlmittelleitungen mit dem im Kühlkörper vorliegenden Kühlmittelpfad verbunden ist. Der Kühlmittelpfad im Kopfteil kann als Bypass zu dem Kühlmittelkreislauf durch den Kühlkörper gestaltet werden, er kann in die Kühlmittelzuleitung oder in die Ableitung integriert sein, oder er kann durch zwei Teilpfade gebildet werden, jeweils einer in Zu- und in der Ableitung.

Kühlmittelanschlussvorrichtungen an dem Kopfteil verbinden die Zufuhr- und Rücklaufabschnitte über Kühlmittelleitungen mit der Kühlmittelquelle und dem Kühlmittelrücklauf. Für den mechanischen Halt des Lampenmoduls sorgt eine mechanische Anschlussvorrichtung zur Verbindung des Kopfteils mit einer entsprechenden Halterung. Sämtliche Anschlussvorrichtungen können bevorzugt als lösbare Steck-, Schraub-, Steckschraub-, Klemmverbindungen oder ähnliches ausgeführt sein, sodass das Lampenmodul einfach von der Stromversorgungs- und/oder Steuerungsleitung bzw. -vorrichtung, den Kühlmittelleitungen und der mechanischen Halterung getrennt und wieder angefügt werden kann. Halterung und Kühlmittelleitung können je nach Anordnung des Lampenmoduls im Reaktor (horizontal oder vertikal) als starre (Rohr-)Verbindungen (beispielsweise im Falle horizontaler Anordnung) ausgeführt sein oder es werden nicht-starre Schläuche für die Kühlmittelleitungen und Metall- oder Kunststoffseile oder Ketten für die Halterung (beispielsweise im Falle vertikaler Anordnung) eingesetzt, sodass die LED-Lampe quasi frei schwingend am Kopfteil aufgehängt ist, was die Handhabung des Lampenmoduls erleichtert.

Ferner ist es möglich, dass nicht nur die Aufteilung eines Fluidpfads in Zulauf- und Rücklaufabschnitte von der Anzahl und Anordnung der LEDs an dem Kühlkörper abhängt; die Anzahl der Fluidpfade - nicht deren Aufteilung - durch den Kühlkörper ist ebenfalls von der Gesamtanzahl der LEDs des Lampenmoduls abhängig, da einerseits ein Fluidpfad nur zu einer vorbestimmten Kühlleistung führt und andererseits alle LEDs möglichst gleichmäßig gekühlt werden sollen. So kann jeweils ein Fluidpfad zur Kühlung einer weiteren vorbestimmten Anzahl an LEDs vorgesehen sein, die eine LED-Kühlgruppe definiert. Diese vorbestimmte Anzahl an LEDs in der Kühlgruppe kann zwar der vorbestimmten Anzahl der auf einer Trägerstruktur angeordneten bzw. mit einer Stromleitungsvorrichtung verbundenen LEDs entsprechen; allerdings wird die vorbestimmte Anzahl an LEDs in der Kühlgruppe - je nach Kühlkapazität - größer sein und vorzugsweise ein Vielfaches der Anzahl an LEDs sein, die mit einer Stromleitungsvorrichtung verbundenen beziehungsweise auf einer Trägerstruktur angeordneten sind. So können die LEDs, die auf mehreren umfänglich benachbarten Trägerstrukturen entlang eines Mantelflächenabschnitts des Kühlkörpers angeordnet sind, durch einen Fluidpfad gekühlt werden, bei dem sich z. B. ein Zufuhrabschnitt in mehrere Rücklaufabschnitte entsprechend der Anordnung der LEDs an der Mantelfläche aufteilt. Sind mehrere Mantelflächenabschnitte entlang der Länge des Kühlkörpers vorgesehen, sodass mehrere Trägerstrukturen nicht nur umfänglich an dem Kühlkörper, sondern auch entlang dessen Länge benachbart angeordnet sein können, so kann erfindungsgemäß vorgesehen sein, dass die Zufuhrabschnitte der Kühlfluidpfade für eine zweite oder weitere LED-Kühlgruppe nicht am Kopfende des Kühlkörpers - wie für eine erste Kühlgruppe - sondern auf einer entsprechenden Höhe entlang der Länge des Kühlkörpers beginnen. D.h., sie sind dem Mantelflächenabschnitt zugeordnet, an dem die Trägerstrukturen mit den LEDs, die eine zweite oder weitere Kühlgruppe bilden, angeordnet sind. Entsprechendes kann auch für die Rücklaufabschnitte weiterer Fluidpfade gelten.

Ferner kann das Lampenmodul einen oder mehrere Temperatursensor(en) aufweisen, der/die auf der Trägerstruktur/den Trägerstrukturen angeordnet und mit der Stromversorgungs- und Steuerungsvorrichtung des Lampenmoduls, die einen Schulzschalter für LEDs umfasst, verbunden ist/sind. Die Stromversorgungs- und Steuerungsvorrichtung kann ferner alternativ oder zusätzlich zumindest einen Regelkreis für die LED-Ansteuerung aufweisen, mit dem gleichartige oder unterschiedliche LEDs dimmbar sind und/oder das Spektrum der emittierten Wellenlängen unterschiedlicher LEDs änderbar ist, um die emittierte Lichtmenge oder die emittierten Wellenlängen dem Reaktionsverlauf im umgebenden Reaktionsraum anzupassen.

Ein erfindungsgemäßer Photoreaktor weist ein Reaktionsgefäß auf, das einen Reaktionsraum zur Durchführung einer photochemischen Reaktion zumindest eines in das Reaktionsgefäß zugeführten oder dort vorgelegten Edukts begrenzt. Ferner umfasst er zumindest ein in dem Reaktionsgefäß angeordnetes erfindungsgemäßes Lampenmodul mit einem für die photochemische Reaktion geeigneten Emissionsspektrum.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine schematische Seitenansicht auf ein erfindungsgemäßes LED-Lampenmodul, bei dem die LED-Lampe in einem doppelwandigen Tauchrohr angeordnet ist,
- **Fig. 2**: eine Draufsicht auf das LED-Lampenmodul aus Fig. 1,
- **Flg. 3**: eine Seitenansicht der LED-Lampe eines erfindungsgemäßen Lampenmoduls mit Anschlussvorrichtungen für elektrischen Anschluss, Halterung sowie Kühlmittelzufuhr und -rücklauf,
- **Fig. 4a**: eine schematische Draufsicht und dazu
- **Fig. 4b**: eine Seitenschnittansicht entsprechend Schnittlinie AA in Fig. 4a auf den LED-Körper eines Lampenmoduls, dessen Kühlkörper einen viereckigen Querschnitt und eine zentrisch angeordnete Kabelkammer aufweist,
- **Fig. 5**: eine Seitenansicht auf eine am Kühlkörper befestigte Trägerstruktur mit LEDs und elektrischer Kontaktierung,
- **Fig. 6a**: eine schematische Draufsicht und
- **Fig. 6b**: eine Seitenaufschnittansicht entsprechend Schnittlinie AA in Fig. 6a auf den LED-Körper eines Lampenmoduls, dessen Kühlkörper aus einem sechseckigen Strangpressprofil mit zentralem Zulaufkanal und sechs symmetrisch darum angeordneten Rücklaufkanälen gebildet wird,
- **Fig. 7a**: eine schematische Draufsicht und
- **Fig. 7b**: eine Seitenaufschnittansicht entsprechend Schnittlinie AA in Fig. 7a auf den LED-Körper eines Lampenmoduls mit einem Kühlkörpervollprofil, bei dem Kabelkammern durch konkave Einschnitten gebildet werden.

Die erfindungsgemäße Vorrichtung bezieht sich auf ein LED-Lampenmodul, das dazu ausgebildet ist, als Tauchstrahler in einem Photoreaktor eingesetzt zu werden, und auf einen mit einem oder mehreren erfindungsgemaßen LED-Lampenmodulen ausgestatteten Photoreaktor.

In der vorliegenden Beschreibung wird auf LEDs als Lichtquelle Bezug genommen. Hierbei sind alle Licht emittierenden Halbleiterbauelemente, auch organische Leuchtdioden (OLEDs), inbegriffen.

Die Bezeichnung LED-Körper meint in der vorliegenden Beschreibung den aus Kühlkörper und den daran angebrachten Trägerstrukturen mit LEDs bestehenden Körper. Mit der Bezeichnung LED-Lampe ist vorliegend die Einheit aus LED-Körper und Kopfteil gemeint. Das LED-Lampenmodul kann zudem weitere Bauteile, wie etwa ein Tauchrohr, das die LED-Lampe umgibt, etc. aufweisen.

Um mit Nieder- und Mitteldruckstrahlern im Bereich der Photochemie konkurrieren zu können und eine hohe Leistungsdichte zu erreichen, kann es erforderlich sein, Module mit einer Vielzahl an LEDs einzusetzen bzw. die LEDs mit hoher Leistung zu betreiben, wodurch eine aktive Kühlung mit einem Kühlmittel, wie z. B. Wasser, erforderlich ist. Wasser als Kühlmittel kann deionisiert sein und/oder Zusätze aufweisen, um Verschmutzungen von Anlagenkomponenten durch Bestandteile des Kühlwassers oder Wachstum von Mikroorganismen zu verhindern. Die Art des Kühlmittels kann auch von der Reaktionstemperatur im umgebenden Reaktionsmedium abhängig sein, so kann z. B. bei Reaktionstemperaturen, die unter 10 °C liegen, als Kühlmittel beispielsweise Glycerin, Glykole oder Alkohole oder nicht leitfähige Silikonöle eingesetzt werden; selbstverständlich auch in Mischungen oder wässriger Lösung. Das Handhaben und Beherrschen des Wärmehaushalts - kurz thermisches Management - eines LED-Lampenmoduls, das auch die Prozesstemperatur des Reaktionsmediums, das das Lampenmodul umgibt, berücksichtigen muss, ist für eine adäquate Lebensdauer von entscheidender Bedeutung.

Im Bereich der industriellen Photochemie werden auch Lampen mit deutlich größeren Abmessungen (bis zu 2 m Länge oder auch länger bei Mitteldruckstrahlern) und entsprechender Leistung eingesetzt, was den Einsatz von LEDs auf diesem Gebiet bislang beschränkt hat, da hier die Anforderungen an Stromzufuhr und auch Kühlung bislang nicht erfüllt wurden - Länge bzw. Anzahl der in Reihe geschalteten LEDs mit hoher Leistungsdichte ist wegen der dadurch ansteigenden Spannung begrenzt; denn die Halbleiterelemente werden mit geringen Spannungen betrieben. Somit stellen die Kapazitätsverluste eine Herausforderung dar, die größere Kabelquerschnitte erfordern und die Leitungslänge beschränken Während es bei Mitteldruckstrahlenquellen aufgrund der problemlos realisierbaren langen Kabellängen von mehreren hundert Metern möglich ist, die Stromversorgung und Steuerung der Lichtquelle außerhalb des Photoreaktors und damit außerhalb einer potentiell explosionsfähigen Umgebung unterzubringen, ist dies bei den LED Lampenmodulen aufgrund der angeführten Beschränkungen nicht möglich.

Eine weitere Schwierigkeit bei längeren Lampenmodulen liegt zudem in der Bereitstellung einer gleichmäßigen Kühlung für alle LEDs, um zu verhindern, dass die LEDs nahe stromaufwärts gelegener Abschnitte des Fluidpfads nicht deutlich stärker gekühlt werden als LEDs, die an weiter stromabwärts gelegenen Abschnitten des Fluidpfads angeordnet sind.

Anders als in Bioreaktoren, in denen üblicherweise einfach zu handhabende Temperatur- und Druckbedingungen im Raumtemperatur und Atmosphärendruckbereich liegen, müssen Lampenmodule, die in photochemischen Reaktoren zum Einsatz kommen, extremeren Temperatur- und Druckbedingungen aushalten können. Werden nun temperaturempfindliche LEDs als Strahlungsquellen eingesetzt, ergeben sich besondere Anforderungen hinsichtlich des thermischen Managements.

Das erfindungsgemäße LED-Lampenmodul ist primär zur industriellen Anwendung in der präparativen Photochemie vorgesehen, und muss daher den erhöhten Anforderungen genügen. Hierzu zählen insbesondere von Raumtemperatur und Umgebungsdruck abweichende Reaktionstemperaturen und -drücke, die im Reaktionsraum um das Lampenmodul herrschen, und die auch Temperaturen unter +5°C und über +40°C sowie Drücke im Bereich von Hochvakuum und 6 bar Überdruck umfassen. Ferner ist bei den in Photoreaktoren eingesetzten Lichtquellen -je nach Zusammensetzung des Reaktionsvolumens - auf den Explosionsschutz zu achten; die erfindungsgemäßen LED-Lampenmodulen können in bestimmten Ausführungsformen auch in ATEX klassifizierten Bereichen eingesetzt werden.

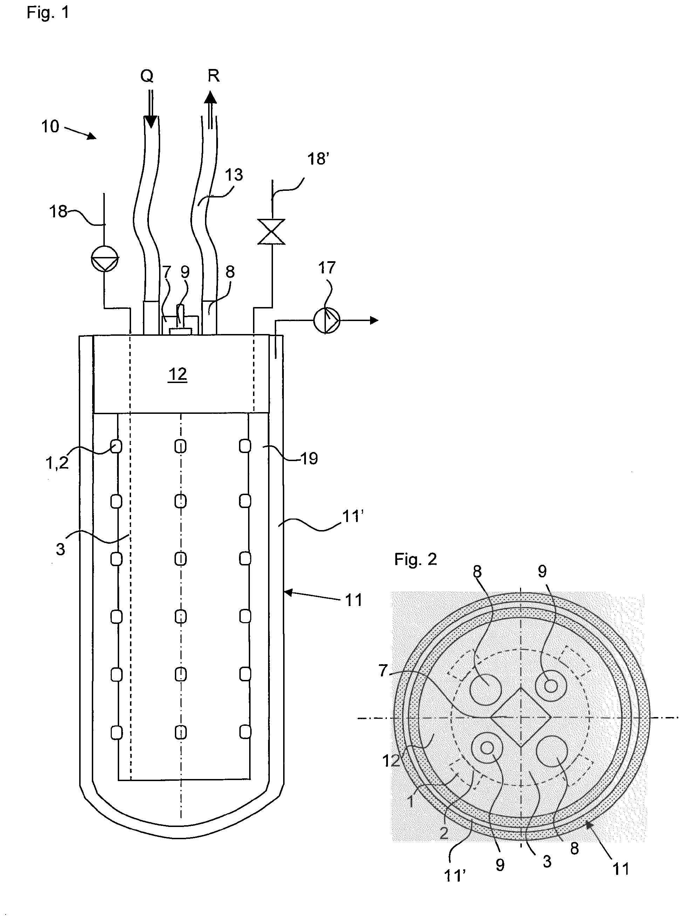

**Fig. 1** zeigt ein erfindungsgemäßes Lampenmodul 10 mit einem doppelwandigen Tauchrohr 11, in dem der so gebildete Spalt 11' mittels einer Pumpe 17 evakuiert werden kann, um die thermische Entkopplung des Tauchrohrinnenraums 19 von dem umgebenden Reaktionsraum zu verbessern. Alternativ dazu - figurativ allerdings nicht dargestellt - ist auch die Spülung des Spalts 11' mit einem Kühlmedium denkbar. Eine Flüssigkühlung kann Wasser oder andere flüssige Medien, eine Gasdurchspülung beispielsweise Luft oder Stickstoff verwenden. Vorteilhaft wird durch das zusätzliche bzw. doppelwandige Tauchrohr die Oberfläche der Tauchlampe vergrößert und damit eine erhöhte photochemische Effizienz realisiert.

Von dieser thermischen Entkopplung unabhängig ist in Fig. 1 ferner das Kopfteil 12 abgedichtet in dem Tauchrohr 11 gelagert, sodass durch Inertgaszufuhr über die Leitung 18, die sich hier durch das Köpfteil 12 und den distanziert angeordneten Kühlkörper 3 erstreckt, im Tauchrohrinneren 19 um den LED Körper eine Inertgasatmosphäre mit einem gewissen Überdruck gegenüber dem atmosphärischen Umgebungsdruck erzeugt werden und so der Explosionsschutz des Lampenmoduls 10 verbessert werden kann. Über die mit Ventil verschließbare Inertgasableitung 18' kann zu Revisionszwecken der Überdruck abgelassen werden, alternativ kann die Inertgaszufuhr- und abfuhr so eingeregelt werden, dass eine Inertgasspülung unter besagtem Überdruck durchgeführt wird. Hierzu strömt das Inertgas unten aus dem Kühlkörper oder durch ein entsprechend angeordnetes Rohr (oder Schlauch) aus und außen an den LEDs vorbei nach oben zu der druckabhängig durchlassventilgesteuerten Inertgasableitung. Durch die Inertgasatmosphäre bzw. Spülung wird der Innenbereich des Tauchrohres inertisiert. Die Aufrechterhaltung kann über einen kontinuierlichen Durchfluss oder eine Leckageausgleichskompensation mit einer entsprechenden Steuer-, Mess- und Regeleinheit (vorzugsweise ATEX zertifiziert) erfolgen. Zum Schutz der empfindlichen Halbleiterbauteile sollte der maximale Überdruck im Tauchrohr allerdings 15 mbar nicht überschreiten. Damit lässt sich die Zündschutzart "p" gem. ATEX Direktive (Überdruckkapselung) realisieren.

Beide Einrichtungen - thermische Entkopplung und Explosionsschutz - können auch unabhängig voneinander für verschiedene Ausführungen des Lampenmoduls eingesetzt werden.

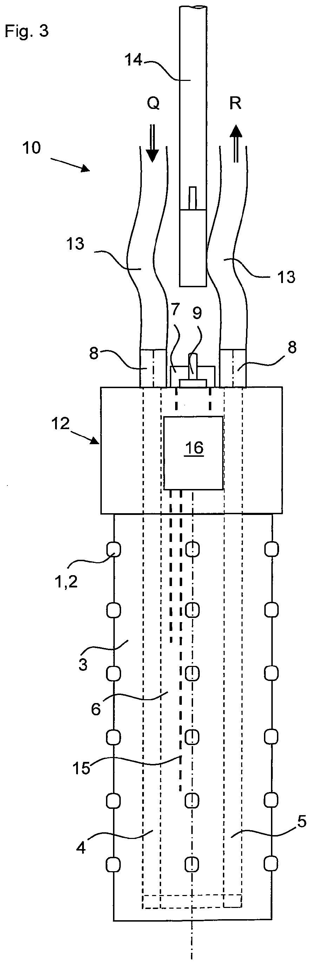

Weiter ist in Fig. 1 am Kopfteil 12 ein Steckanschluss 7 zur Herstellung der elektrischen Kontaktierung der LEDs 1 und ein Steckanschluss 9 zur Aufhängung des Lampenmoduls 10 mittels einer entsprechend ausgestatteten Halterung (siehe Flg. 3) zu sehen. Über Kühlmittelanschlussvorrichtungen 8 wird der im Inneren des Kühlkörpers 3 vorliegende Kühlfluidpfad über Kühlmittelleitungen 13 mit einer Kühlmittelquelle Q und einem Kühlmittelrücklauf R verbunden. Dabei kann es sich auch um einen Kühlmittelkreislauf handeln. Dem Kühlmittel wie z. B. Wasser kann auch ein Mittel zur Verhinderung von Keimwachstum zugesetzt werden.

**Fig. 2** zeigt die mit dem Kopfteil 12 dicht in dem doppelwandigen Tauchrohr 11 angeordnete LED-Lampe von oben. Das Material des Tauchrohrs 11 ist so gewählt, dass es für die von den LEDs 1 emittierte Strahlung transparent ist und dem Reaktionsdruck im umgebenden Reaktionsraum standhält. Filter für unerwünschte Wellenlängen wie bei herkömmlichen Strahlungsquellen sind nicht erforderlich, da die gewünschte Wellenlänge durch entsprechende Dotierung der LEDs 1 eingestellt werden kann. In dem in Fig. 2 gezeigten Beispiel liegt der elektrische Anschluss 7 axial zentral in Bezug auf den Kühlkörper 3, die Kühlmittelanschlüsse 8 und Steckverbinder 9 für die Halterung sind konzentrisch um den elektrischen Anschluss 7 verteilt.

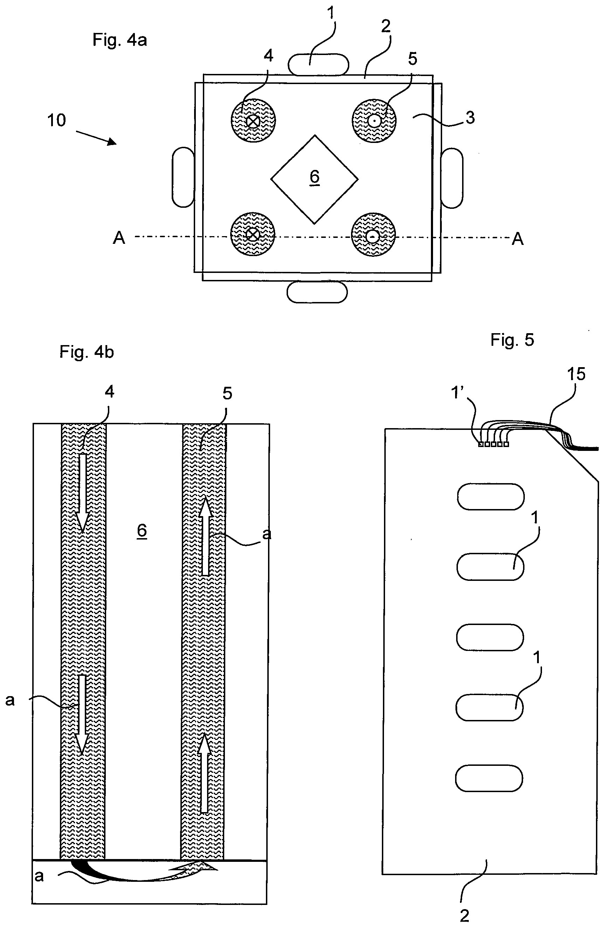

In **Fig. 3** ist ebenfalls ein LED-Körper mit Kopfteil 12 eines LED-Lampenmoduls 10 gezeigt, bei dem LED-Chips, die jeweils eine LED 1 auf einer Trägerstruktur 2 umfassen, an der Außenseite eines Kühlkörpers 3 angeordnet sind. Am Kopfteil des Kühlkörpers 3 sind lösbare Schlauchanschlüsse 8 zur Verbindung des im Inneren des Kühlkörpers 3 vorliegenden Fluidpfads (siehe **Fig. 4a****,b; 6a,b** und **7a,b**) mit einer Kühlmittelquelle Q und einem Kühlmittelrücklauf R vorgesehen. In der gezeigten Ausführungsform umfasst das Kopfteil 12 auch die Stromversorgungs- und Steuervorrichtung 16, die von dem gleichen Kühlkreislauf wie der Fluidpfad in dem Kühlkörper 3 gekühlt wird, indem Fluidpfadabschnitte durch das Kopfteil 12 verlaufen. Da die Leistungskomponenten der LED-Lampe wie Treiber und Netzteile ebenso Halbleiterbauteile beinhalten, die temperaturempfindlich sind, kann die für die LEDs verwendete Kühlung ebenso dazu verwendet werden, um die Leistungselektronik bzw. die LED Treiber im Kopfteil durch den gleichen Kühlwasserkreislauf zu kühlen.

Figuren **4a****,b; 6a,b** und **7a,b** zeigen jeweils verschiedene Ausführungsformen eines erfindungsgemäßen Lampenmoduls 10 in a) Querschnitt- und b) Längsschnittansicht.

**Fig. 4a,b** zeigt einen Kühlkörper 3 mit viereckigem Querschnitt, bei dem auf den Außenflächen Trägerplatinen 2 mit LEDs 1 angebracht sind. In diesem Beispiel liegt die Kabelkammer 6 axial-zentral, zwei Kühlzulaufabschnilte 4 und zwei Kühlrücklaufabschnitte 5 sind darum verteilt angeordnet.

Selbstverständlich sind Abweichungen von Anzahl und Anordnung der Kühlmittelzuläufe und Rückläufe im Schutzumfang der Erfindung enthalten und hängen von Querschniltsform und Länge des Lampenmoduls bzw. des Kühlkörpers ab.

Da der Abstrahlwinkel herkömmlicher LEDs beschränkt ist, sind mehreckige, z. B. sechs- oder achteckige Profilquerschnitte vorteilhaft. Werden OLEDs eingesetzt, können auch kreiszylindrische Formen gut realisiert werden.

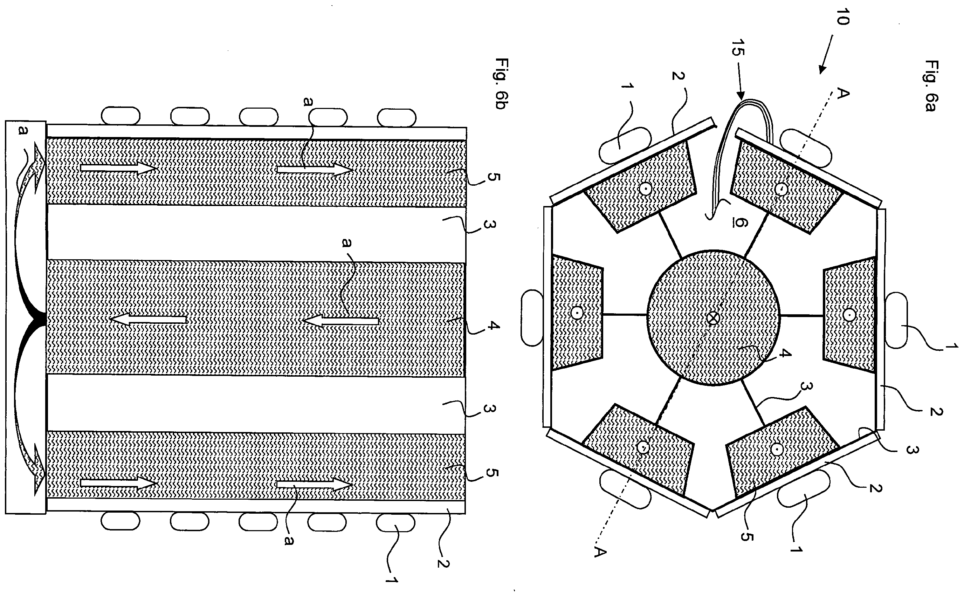

**Fig. 5** zeigt eine Trägerstruktur 2, auf der fünf LEDs 1 angeordnet sind, die in Reihe geschaltet durch in einer Kabelkammer untergebrachte elektrische Leitungen 15' an den auf der Trägerstruktur 2 vorgesehenen Kontaktelementen 1' kontaktiert sind. Dabei ist eine Ecke der rechteckigen Trägerstruktur 2 ausgespart, um die elektrischen Leitungen 15' nach innen in die Kabelkammer führen zu können, wie dies auch in **Fig. 6a** angedeutet ist.

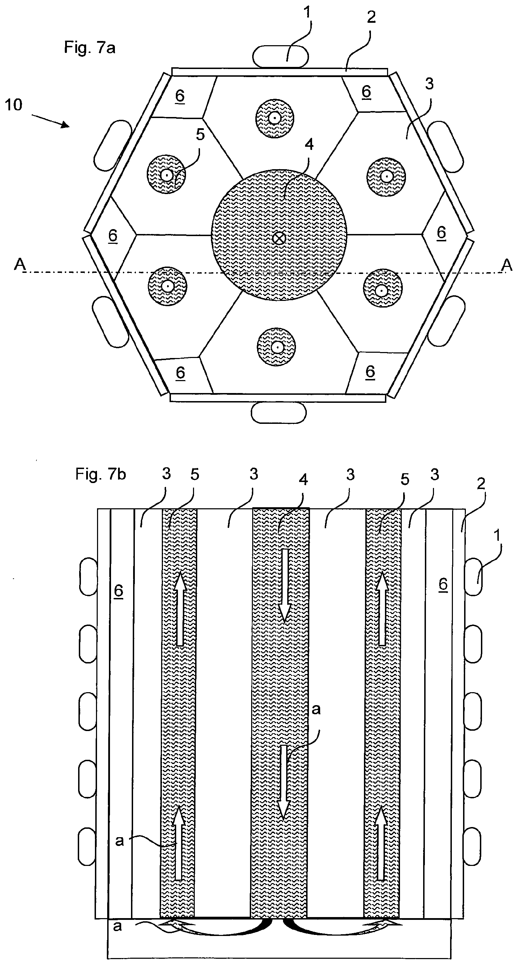

Den Lampenmodulen 10 in **Fig. 6a,b** und **7a,b** ist gemeinsam, dass der Zufuhrabschnitt 4 des Fluidpfads a, der im Einsatz mit der Kühlmittelquelle verbunden wird, axial zentral in dem Kühlkörper 3 verläuft. Die Rücklaufabschnitte 5 sind axialparallel und konzentrisch um den Zufuhrabschnitt 4 im Kühlkörper 3 angeordnet. Dabei entspricht die Anordnung der Rücklaufabschnitte 5 in dem Kühlkörper 3 der Anordnung der LEDs 1, die mit den Trägerstrukturen 2 an den ebenen, d. h. nicht-konkaven Abschnitten der Mantelfläche des prismatischen Kühlkörpers 3 angeordnet sind.

Die Ausführungsformen in **Fig. 6a,b** und **7a,b** unterscheiden sich allerdings in der Art des Kühlkörpers 3 und in der Anordnung der Kabelkammer 6.

Das LED-Lampenmodul 10 in **Fig. 6a,b** wird mit einem sechseckigen Hohlprofil, beispielsweise aus Aluminium mittels Strangpressen, als Kühlkörper 3 mit innenliegenden Zufuhr- und Rücklaufkanälen 4,5 erhalten. In dem Hohlprofil liegen im vorliegenden Fall sechs Rücklaufkanäle 5 vor. Axial-zentral liegt ein Zulaufkanal 4 vor, der über Stege mit den Rücklaufkanälen 5 verbunden ist, sodass die Zwischenräume zwischen Zulaufkanal 4 und Rücklaufkanälen 5 als Kabelkammern 6 zur Verfügung steht.
**Fig. 7a,b** zeigt ein Lampenmodul mit einem Kühlkörper 3, der wie der Kühlkörper in **Flg. 4a,b** als Vollprofil (aus Aluminium) ausgeführt ist, das hier jedoch auf einem sechseckigen Querschnitt basiert, bei dem die Kanten konkav eingeschnitten sind, so dass es sich bei der Grundfläche tatsächlich um ein 18-Eck handelt. In diesen konkaven Einschnitten wird jeweils eine Kabelkammer 6 untergebracht, die durch die an dem Kühlkörper 3 befestigten Trägerstrukturen 2 geschlossen werden. Kühlmittelzulauf 4 und Rückläufe 5 werden durch in das Vollprofil eingebrachte Bohrungen gebildet. Alternativ kann der Kühlkörper 3 auch in der gewünschten Weise gegossen werden, gegebenenfalls auch mehrstückig.

Generell kann ein erfindungsgemäßes LED-Lampenmodul zumindest einen Temperatursensor auf der Trägerstruktur bzw. der Platine aufweisen, welche für eine Schutzabschaltung zum Schutz der Halbleiterbauteile sorgt, sofern die maximal zulässige Umgebungstemperatur überschritten wird. Bei Unterschreiten der Maximaltemperatur kann die Steuerungsvorrichtung die entsprechenden LEDs wieder selbsttätig zuschalten. Sämtliche sicherheilsrelevante Sensoren des Lampenmoduls können redundant bzw. zweikanalig ausgeführt sein, um die entsprechend notwendige SIL Klasse zu realisieren.

Da die LEDs gruppenweise auf den Trägerstrukturen untergebracht sind, können diese bei Ausfall einzeln ausgetauscht werden.

Ferner ist das erfindungsgemäße Lampenmodul geeignet, prozessspezifische Spektren bereitzustellen, wobei ferner mittels Regelkreis die Strahlungsintensität dem Photochemischen Prozess angepasst werden kann. So kann eine Leistungsregelung der LEDs (Dimming) zur Prozesskontrolle erfolgen, da in vielen Reaktionen sich die Absorption während des Prozesses ändert. Hierauf kann durch gezielte Mess- und Regelkreise und Lampendimming reagiert werden, um ein effizientes System zu realisieren und Überbestrahlungen zu vermeiden.

Ein erfindungsgemäßes Lampenmodul kann monochromatische LEDs sowie eine Mischung von LEDs mit unterschiedlichen Emissionsspektren aufweisen, welche die optimale Ausnutzung des Absorptionsspektrums der jeweiligen Reaktion darstellt. Gleiches gilt, wenn die Tauchlampen für Bioreaktoren eingesetzt werden sollen. Hier können LEDs mit unterschiedlichen Emissionswellenlängen auf einer Trägerstruktur implementiert werden, um optimale Wachstumsraten zu erzielen. In unterschiedlichen Wachstumsphasen bzw. unterschiedlichen Zellen können jeweils optimal gemischte Lichtspektren und - intensitäten zum optimierten Wachstum bereitgestellt werden.

## Patentansprüche

1. Lampenmodul (10) für photochemische Reaktoren, das einen Kühlkörper (3), an dessen Außenseite zumindest eine Trägerstruktur (2) mit zumindest einer Licht emittierenden Diode (LED) (1) angeordnet ist, und
ein Kopfteil (12) zum elektrischen Anschluss der zumindest einen LED (1) und zur Halterung des Lampenmoduls (10) aufweist,
wobei der Kühlkörper (3) zumindest einen Fluidpfad (a) begrenzt, der zumindest einen Zufuhrabschnitt (4) und zumindest einen Rücklaufabschnitt (5) aufweist, wobei der zumindest eine Zufuhrabschnitt (4) über das Kopfteil (12) mit einer Kühlmittelquelle (Q) und der zumindest eine Rücklaufabschnitt (5) über das Kopfteil (12) mit einem Kühlmittelrücklauf (R) fluidisch verbunden ist,
**dadurch gekennzeichnet, dass**
das Lampenmodul (10)
- zumindest zwei ineinander angeordnete Tauchrohre oder
- zumindest ein doppelwandiges Tauchrohr (11)
aus einem für die Wellenlängen der von den LEDs (1) des Lampenmoduls (10) emittierten Strahlung durchlässigen Material aufweist,
in denen/in dem zumindest der Kühlkörper (3) angeordnet ist, an dem die zumindest eine Trägerstruktur (2) mit der zumindest einen Licht emittierenden Diode (LED) (1) angeordnet ist,
wobei ein zwischen den zwei Tauchrohren oder den zwei Wänden des doppelwandigen Tauchrohrs (11) gebildeter Spalt (11') eine thermische Entkopplung bereitstellt
und wobei der Kühlkörper (3) zumindest eine Kammer (6) zumindest teilweise begrenzt, durch die sich die zumindest eine Stromversorgungs- und/oder Steuerungsleitung (15) von dem Kopfende des Kühlkörpers (3) bis zu einem Kontaktelement (1') der zumindest einen LED (1) erstreckt.

2. Lampenmodul (10) nach Anspruch 1,
**dadurch gekennzeichnet, dass**
die ineinander angeordneten Tauchrohre oder das doppelwandige Tauchrohr (11) bezüglich Material und Form für einen Reaktionsdruck in einem das Tauchrohr (11) umgebenden Reaktionsraum für einen Bereich von Hochvakuum bis 6 bar Überdruck ausgelegt sind/ist, und/oder
das Kopfteil (12) mit einem ersten Ende des Tauchrohrs (11) fluiddicht und federnd gelagert verbunden ist,
und dass das Tauchrohr (11) an seinem zweiten Ende geschlossen ist, bevorzugt durch ein Verschlussteil, wobei
sich ein Inertgaszufuhrkanal durch das Kopfteil (12) in den Raum (19) zwischen dem Tauchrohr (11) und dem Kühlkörper (3) erstreckt.

3. Lampenmodul (10) nach Anspruch 1 oder 2,
**dadurch gekennzeichnet, dass**
eine vorbestimmte Anzahl an LEDs (1), die bevorzugt auf einer gemeinsamen Trägerstruktur (2) angeordnet sind, jeweils mit zumindest einer der Stromversorgungs- und/oder Steuerungsleitungen (15) verbunden ist und wobei eine Anzahl und Größe der Kammer(n) (6) geeignet ist, die Gesamtheit der LEDs (1) des Lampenmoduls (10) aufzunehmen und von einer Gesamtanzahl der LEDs des Lampenmoduls und deren Verteilung entlang der Kühlkörpermantelfläche abhängig ist, denn mit zunehmender LED-Zahl wächst der erforderliche Kabelquerschnitt infolge der bei Reihenschaltung von Halbleiterbauteilen auftretenden Kapazitätsverluste, die mit geringer Spannung betrieben werden.

4. Lampenmodul (10) nach zumindest einem der Ansprüche 1 bis 3,
**dadurch gekennzeichnet, dass**
der Kühlkörper (3) entsprechend der Anzahl und Anordnung der LEDs (1) auf der zumindest einen Trägerstuktur (2) dimensioniert ist.

5. Lampenmodul (10) nach zumindest einem der Ansprüche 1 bis 4,
**dadurch gekennzeichnet, dass**
der Kühlkörper (3) zumindest einen längsaxial und zentral verlaufenden Zufuhrabschnitt (4) und mehrere Rücklaufabschnitt (5) aufweist, die axialparallel um den Zufuhrabschnitt (4) angeordnet sind,
oder
die Kammer (6) längsaxial und zentral in dem Kühlkörper (3) verläuft.

6. Lampenmodul (10) nach Anspruch 5,
**dadurch gekennzeichnet, dass**
bei dem längsaxial und zentral verlaufenden Zufuhrabschnitt (4) die Kammer (6) konzentrisch um den längsaxial und zentral verlaufenden Zufuhrabschnitt (4) angeordnet ist, oder
mehrere axialparallel verlaufende Kammern (6)
- innerhalb des Kühlkörpers (3) vorgesehen sind oder
- durch Einschnitte des Kühlkörpers (3) an dessen Außenseite in Verbindung mit der zumindest einen an dem Kühlkörper (3) angeordneten Trägerstruktur (2) gebildet werden.

7. Lampenmodul (10) nach zumindest einem der Ansprüche 1 bis 6,
**dadurch gekennzeichnet, dass**
an dem Kopfteil (12) zumindest eine elektrische Anschlussvorrichtung (7) zur Verbindung der zumindest einen Stromversorgungs- und/oder Steuerungsleitung (15) mit einer Stromversorgungs- und Steuerungsvorrichtung (16) des Lampenmoduls (10) vorgesehen ist, oder
das Kopfteil (12) die Stromversorgungs- und Steuerungsvorrichtung (16) für die zumindest eine LED (1) enthält, und zumindest einen Kühlmittelpfad zur Kühlung der Stromversorgungs- und Steuerungsvorrichtung aufweist, der vorzugsweise mit dem im Kühlkörper (3) vorliegenden Kühlmittelpfad (a) verbunden ist, und
an dem Kopfteil (12)
- Kühlmittelanschlussvorrichtungen (8) zur Verbindung der Zufuhr- und Rücklaufabschnitte (4,5) über Kühlmittelleitungen (13) mit der Kühlmittelquelle (Q) und dem Kühlmittelrücklauf (R), und
- zumindest eine mechanische Anschlussvorrichtung (9) zur Verbindung des Kopfteils (12) mit einer Halterung (14) vorliegen.

8. Lampenmodul (10) nach zumindest einem der Ansprüche 1 bis 7,
**dadurch gekennzeichnet, dass**
die Anzahl der Fluidpfade (a), die sich durch den Kühlkörper (3) erstrecken, in Bezug auf eine vorbestimmte Kühlleistung, die für die Gesamtanzahl der LEDs (1) des Lampenmoduls (10) zu erbringen ist, ausgewählt ist,
wobei jeweils ein Fluidpfad (a) zur Kühlung einer vorbestimmten Anzahl an LEDs (1) vorgesehen ist, die eine Kühlgruppe definiert,
wobei der zumindest eine Zufuhrabschnitt (4) des Fluidpfads (a) für eine erste Kühlgruppe am Kopfende des Kühlkörpers (3) und jeder weitere Zufuhrabschnitt (4) für einen Fluidpfad (a) einer weiteren Kühlgruppe
auf einer Höhe entlang der Länge des Kühlkörpers (3) beginnen, die einem Mantelflächenabschnitt des Kühlkörpers (3) entspricht, an dem die LEDs (1) jeder weiteren Kühlgruppe angeordnet sind.

9. Lampenmodul (10) nach Anspruch 7 oder 8,
**dadurch gekennzeichnet, dass**
das Lampenmodul (10) zumindest einen Temperatursensor aufweist, der auf der Trägerstruktur (2) angeordnet ist und mit der Stromversorgungs- und Steuerungsvorrichtung (16), die einen Schutzschalter für die LEDs (1) umfasst, verbunden ist, und/oder dass
- die Stromversorgungs- und Steuerungsvorrichtung (16) zumindest einen Regelkreis für eine LED-Ansteuerung aufweist, mit dem gleichartige oder unterschiedliche LEDs (1) dimmbar sind und/oder das Spektrum der emittierten Wellenlängen unterschiedlicher LEDs (1) änderbar ist.

10. Photoreaktor mit einer darin angeordneten Lampe mit einem für die photochemische Reaktion geeigneten Emissionsspektrum,
**dadurch gekennzeichnet, dass**
die Lampe ein Lampenmodul (10) nach zumindest einem der Ansprüche 1 bis 9 ist.

## Claims

1. Lamp module (10) for photochemical reactors comprising a cooling body (3), which has at least one carrier structure (2) with at least one light-emitting diode (LED) (1) arranged on its outside, and a head part (12) for electrical connection of the at least one LED (1) and for mounting the lamp module (10), whereby the cooling body (3) borders at least one fluid path (a) that comprises at least one feed section (4) and at least one return section (5), whereby the at least one feed section (4) is fluidically connected to a coolant source (Q) via the head part (12) and the at least one return section (5) is fluidically connected to a coolant return (R) via the head part (12),
**characterised in that**
the lamp module (10) comprises
- at least two immersion tubes that are arranged one in the other, or
- at least one double-walled immersion tube (11)
made of a material that is transmissive to the wavelengths of the radiation emitted by the LEDs (1) of the lamp module (10),
in which is arranged at least the cooling body (3), at which the at least one carrier structure (2) with the at least one light-emitting diode (LED) (1) is arranged,
whereby a gap (11') is generated between the two immersion tubes or the two walls of the double-walled immersion tube (11) provides for a thermal decoupling
and whereby the cooling body (3) borders, at least in part, at least one chamber (6) through which extends the at least one power supply cable and/or control cable (15) from the head end of the cooling body (3) to a contact element (1') of the at least one LED (1).

2. Lamp module (10) according to claim 1,
**characterised in that**
the immersion tubes that are arranged one in the other or the double-walled immersion tube (11) are/is designed, in terms of material and shape, for a reaction pressure in a reaction space surrounding the immersion tube (11) ranging from high vacuum up to 6 bar overpressure and/or
the head part (12) is connected to a first end of the immersion tube (11) in a fluid-tight manner and such as to be supported in a spring-like manner,
and **in that** the immersion tube (11) is closed on its second and, preferably by means of a closure part, whereby
an inert gas feed channel extends through the head part (12) into the space (19) between the immersion tube (11) and the cooling body (3).

3. Lamp module (10) according to claim 1 or 2,
**characterised in that**
a predetermined number of LEDs (1), which preferably are arranged on a common carrier structure (2), each is connected to at least one of the power supply cables and/or control cables (15), and whereby a number and size of the chamber(s) (6) is suitable for accommodating the entirety of the LEDs (1) of the lamp module (10) and is dependent on a total number of the LEDs of the lamp module and their distribution along the cooling body jacket surface, since the requisite cable cross-section increases with increasing number of LEDs due to the losses of capacitance in a serial connection of semiconductor components operated at low voltage.

4. Lamp module (10) according to at least one of the claims 1 to 3,
**characterised in that**
the cooling body (3) is dimensioned according to the number and arrangement of the LEDs (1) on the at least one carrier structure (2).

5. Lamp module (10) according to at least one of the claims 1 to 4,
**characterised in that**
the cooling body (3) comprises at least one feed section (4) extending longitudinal-axial and central, and multiple return sections (5) that are arranged axial-parallel about the feed section (4),
or
the chamber (6) extends longitudinal-axially and centrally in the cooling body (3).

6. Lamp module (10) according to claim 5,
**characterised in that,**
referring to the feed section (4) extending longitudinal-axial and central, the chamber (6) is arranged such as to be concentric about the feed section (4) that extends longitudinal-axial and central, or
multiple chambers (6) that extend axial-parallel
- are provided inside the cooling body (3) or
- are formed by incisions of the cooling body (3) on its outside in connection with the at least one carrier structure (2) that is arranged on the cooling body (3).

7. Lamp module (10) according to at least one of the claims 1 to 6,
**characterised in that**
at least one electrical connecting device (7) for connecting the at least one power supply cable and/or control cable (15) to a power supply device and control device (16) of the lamp module (10) is provided on the head part (12), or
the head part (12) contains the power supply device and control device (16) for the at least one LED (1), and comprises at least one coolant path for cooling of the power supply device and control device, which preferably is connected to the coolant path (a) present in the cooling body (3), and
- coolant connecting devices (8) for connecting the feed and return sections (4, 5) by means of coolant conduits (13) to the coolant source (Q) and the coolant return (R) are situated on the head part (12), and
- at least one mechanical connecting device (9) for connecting the head part (12) to a mound (14) is present.

8. Lamp module (10) according to at least one of the claims 1 to 7,
**characterised in that**
the number of fluid paths (a) that extend through the cooling body (3) is selected with reference to a predetermined cooling power that is to be provided for the total number of LEDs (1) of the lamp module (10),
whereby one fluid path (a) each is provided for cooling a predetermined number of LEDs (1) that define a cooling assembly,
whereby the at least one feed section (4) of the fluid path (a) for a first cooling assembly originates on the head end of the cooling body (3) and each further feed section (4) for a fluid path (a) of a further cooling assembly originates at one level along the length of the cooling body (3), which corresponds to a jacket surface section of the cooling body (3), on which the LEDs (1) of each further cooling assembly are arranged.

9. Lamp module (10) according to claim 7 or 8,
**characterised in that**
the lamp module (10) comprises at least one temperature sensor that is arranged on the carrier structure (2) and is connected to the power supply device and control device (16) that comprises a circuit breaker for the LEDs (1), and/or **in that**
- the power supply device and control device (16) comprises at least one control loop for an LED triggering by means of which identical or different LEDs (1) can be dimmed and/or the spectrum of the emitted wavelengths of different LEDs (1) can be changed,

10. Photoreactor with a lamp with a suitable emission spectrum for the photochemical reaction arranged in it,
**characterised in that**
the lamp is a lamp module (10) according to at least one of the claims 1 to 9.

## Revendications

1. Module de lampe (10) pour réacteurs photochimiques qui présente un dissipateur thermique (3) sur le côté externe duquel au moins une structure porteuse (2) est disposée avec au moins une diode électroluminescente (LED) (1), et
une partie de tête (12) pour le raccord électrique de l'au moins une LED (1) et pour la fixation du module de lampe (10),
dans lequel le dissipateur thermique (3) limite au moins une voie fluidique (a) qui présente au moins une section d'amenée (4) et au moins une section de retour (5), dans lequel l'au moins une section d'amenée (4) est connectée de manière fluidique par le biais de la partie de tête (12) à une source de réfrigérant (Q) et l'au moins une section de retour (5) est connectée de manière fluidique par le biais de la partie de tête (12) à un retour de réfrigérant (R),
**caractérisé en ce que**
le module de lampe (10) présente
- au moins deux tubes plongeurs disposés l'un dans l'autre ou
- au moins un tube plongeur à double paroi (11)
en un matériau perméable pour les longueurs d'onde du rayonnement émis par les LED (1) du module de lampe (10),
dans le(s)quel(s) au moins le dissipateur thermique (3) est disposé, sur lequel l'au moins une structure porteuse (2) est disposée avec l'au moins une diode électroluminescente (LED) (1),
dans lequel une fente (11') formée entre les deux tubes plongeurs ou les deux parois du tube plongeur à double paroi (11) fournit un découplage thermique et dans lequel le dissipateur thermique (3) limite au moins partiellement au moins une chambre (6) à travers laquelle l'au moins une ligne d'alimentation électrique et/ou de commande (15) s'étend de l'extrémité de tête du dissipateur thermique (3) jusqu'à un élément de contact (1') de l'au moins une LED (1).

2. Module de lampe (10) selon la revendication 1,
**caractérisé en ce que**
les tubes plongeurs disposés l'un dans l'autre ou le tube plongeur à double paroi (11) est/sont conçu(s) en ce qui concerne matériau et forme pour une pression réactionnelle dans un espace réactionnel entourant le tube plongeur (11) pour une région de vide élevé jusqu'à 6 bars de surpression, et/ou
la partie de tête (12) est connectée de manière étanche au fluide et logée de manière élastique à une première extrémité du tube plongeur (11),
et que le tube plongeur (11) est fermé à sa seconde extrémité, de préférence par une partie de fermeture, dans lequel
un canal d'amenée de gaz inerte s'étend à travers la partie de tête (12) dans l'espace (19) entre le tube plongeur (11) et le dissipateur thermique (3).

3. Module de lampe (10) selon la revendication 1 ou 2,
**caractérisé en ce que**
un nombre prédéterminé de LED (1) qui sont de préférence disposées sur une structure porteuse commune (2), est à chaque fois connecté à au moins une des lignes d'alimentation électrique et/ou de commande (15) et dans lequel un nombre et une taille de la/des chambre(s) (6) conviennent à recevoir l'ensemble des LED (1) du module de lampe (10) et dépendent d'un nombre total des LED du module de lampe et de leur répartition le long de la surface d'enveloppe du dissipateur thermique, car avec un nombre croissant de LED croît la section transversale de câble nécessaire suite aux pertes de capacité survenant lors du montage en série de composants à semiconducteur qui sont exploités avec une faible tension.

4. Module de lampe (10) selon au moins une des revendications 1 à 3,
**caractérisé en ce que**
le dissipateur thermique (3) est dimensionné en fonction du nombre et de l'agencement des LED (1) sur l'au moins une structure porteuse (2).

5. Module de lampe (10) selon au moins une des revendications 1 à 4,
**caractérisé en ce que**
le dissipateur thermique (3) présente au moins une section d'amenée (4) s'étendant de manière axiale longitudinalement et centrale et plusieurs sections de retour (5) qui sont disposées de manière parallèle axialement autour de la section d'amenée (4),
ou
la chambre (6) s'étend de manière axiale longitudinalement et centrale dans le dissipateur thermique (3).

6. Module de lampe (10) selon la revendication 5,
**caractérisé en ce que**
en ce qui concerne la section d'amenée (4) s'étendant de manière axiale longitudinalement et centrale,
la chambre (6) est disposée de manière concentrique autour de la section d'amenée (4) s'étendant de manière axiale longitudinalement et centrale, ou
plusieurs chambres (6) s'étendant de manière parallèle axialement
- sont prévues au sein du dissipateur thermique (3) ou
- sont formées par des entailles du dissipateur thermique (3) sur son côté externe en connexion à l'au moins une structure porteuse (2) disposée sur le dissipateur thermique (3).

7. Module de lampe (10) selon au moins une des revendications 1 à 6,
**caractérisé en ce que**
au moins un dispositif de raccord électrique (7) pour la connexion de l'au moins une ligne d'alimentation électrique et/ou de commande (15) à un dispositif d'alimentation électrique et de commande (16) du module de lampe (10) est prévu sur la partie de tête (12), ou
la partie de tête (12) contient le dispositif d'alimentation électrique et de commande (16) pour l'au moins une LED (1), et présente au moins une voie de réfrigérant pour le refroidissement du dispositif d'alimentation électrique et de commande qui est de préférence connectée à la voie de réfrigérant (a) présente dans le dissipateur thermique (3), et
- des dispositifs de raccord de réfrigérant (8) pour la connexion des sections d'amenée et de retour (4, 5) par le biais de conduites de réfrigérant (13) à la source de réfrigérant (Q) et au retour de réfrigérant (R), et
- au moins un dispositif de raccord mécanique (9) pour la connexion de la partie de tête (12) à une fixation (14) sont présents sur la partie de tête (12).

8. Module de lampe (10) selon au moins une des revendications 1 à 7,
**caractérisé en ce que**
le nombre des voies fluidiques (a) qui s'étendent à travers le dissipateur thermique (3) est sélectionné en fonction d'une puissance de refroidissement prédéterminée qui est à fournir pour le nombre total des LED (1) du module de lampe (10),
dans lequel une voie fluidique (a) pour le refroidissement d'un nombre prédéterminé de LED (1) qui définit un groupe de refroidissement est à chaque fois prévue,
dans lequel l'au moins une section d'amenée (4) de la voie fluidique (a) pour un premier groupe de refroidissement à l'extrémité de tête du dissipateur thermique (3) et chaque section d'amenée supplémentaire (4) pour une voie fluidique (a) d'un groupe de refroidissement supplémentaire
commencent à une hauteur le long de la longueur du dissipateur thermique (3) qui correspond à une section de surface d'enveloppe du dissipateur thermique (3) au niveau de laquelle les LED (1) de chaque groupe de refroidissement supplémentaire sont disposées.

9. Module de lampe (10) selon la revendication 7 ou 8,
**caractérisé en ce que**
le module de lampe (10) présente au moins un capteur de température qui est disposé sur la structure porteuse (2) et est connecté au dispositif d'alimentation électrique et de commande (16) qui comprend un disjoncteur de protection pour les LED (1), et/ou que
- le dispositif d'alimentation électrique et de commande (16) présente au moins un circuit de régulation pour une excitation de LED avec lequel des LED (1) de même type ou différentes peuvent subir une variation d'intensité et/ou le spectre des longueurs d'onde émises de différentes LED (1) peut être modifié.

10. Photoréacteur avec une lampe disposée en son sein avec un spectre d'émission convenant à la réaction photochimique,
**caractérisé en ce que**
la lampe est un module de lampe (10) selon au moins une des revendications 1 à 9.
